# EUROPÄISCHE PATENTSCHRIFT

(11) **EP 1 893 987 B1**
(45) Veröffentlichungstag und Bekanntmachung des Hinweises auf die Patenterteilung: **31.12.2008**
(21) Anmeldenummer: 06762075.7
(22) Anmeldetag: 19.06.2006
(51) Int. Cl.: G01N 25/52

(54) **APPARATUR UND VERFAHREN ZUR AUTOMATISIERTEN CETANZAHLBESTIMMUNG**
APPARATUS AND METHOD FOR THE AUTOMATIC DETERMINATION OF THE CETANE NUMBER
APPAREIL ET PROCEDE DE DETERMINATION AUTOMATIQUE DE L'INDICE DE CETANE

(30) Priorität: 20.06.2005 DE 102005028706
(43) Veröffentlichungstag der Anmeldung: 05.03.2008
(73) Patentinhaber: Wilharm, Peter, Dr., 86356 Neusäss-Täfertingen (DE)
(72) Erfinder: Wilharm, Peter, Dr., 86356 Neusäss-Täfertingen (DE)
(74) Vertreter: Gallo, Wolfgang
(86) Internationale Anmeldenummer: PCT/EP2006/005842
(87) Internationale Veröffentlichungsnummer: WO 2006/136349

(56) Entgegenhaltungen:
- WO-A-02/14456
- DE-A1- 19 701 288
- DE-A1- 19 712 921
- US-A- 4 010 358
- US-A- 5 457 985
- "ASTM D 6890 - 04" ASTM DESIGNATION, ASTM INTERNATIOANL, US, Mai 2004 (2004-05), XP008068567

## Beschreibung

Die Erfindung betrifft ein Verfahren und eine Apparatur zur Cetanzahlbestimmung von Mitteldestillaten und biogenen Dieselkraftstoffen und Mischungen daraus.

Die Cetanzahl ist ein Maß für die Zündwilligkeit von Mitteldestillaten, insbesondere Dieselkraftstoff.

Beispiele für Mitteldestillate sind Raffinerieprodukte mit einem Siedebereich von ca. 150 bis 500°C, die als Heizöle, Dieselkraftstoff, Kerosin, Jetfuel erhältlich sind. Beispiele für biogene Dieselkraftstoffe sind Fettsäuremethylester, hergestellt aus Rapsöl, Sojaöl, Palmöl, Rindertalg, Sonnenblumenöl, Altspeisefett und Mischungen daraus.

Eine gute Zündwilligkeit eines Dieselkraftstoffs bedeutet gutes Startverhalten, ruhiger Lauf des Dieselmotors und günstige Abgaswerte.

Die Cetanzahl wird in der Norm EN ISO 5165 und in der Norm ASTM D6890-04 definiert und deren Bestimmung beschrieben. Die Bestimmung von Cetanzahlen erfolgt üblicherweise durch Verwendung eines genormten Viertakt-Einzylinder-Motors mit variablem Verdichtungsverhältnis und indirekter Einspritzung. Da das motorische Verfahren zeitaufwendig und teuer ist, hat es Versuche gegeben, die Cetanzahl mittels Konstantvolumen-Brennkammern ("Constant Volume Combustion Chamber Method") zu bestimmen.

Bei einer Konstantvolumen-Apparatur zur Cetanzahlbestimmung besteht ein Messzyklus aus folgenden Arbeitsschritten:
1. Befüllung einer Brennkammer mit Druckluft und Aufheizung der Brennkammer,
2. Kraftstoffeinspritzung mittels Einspritzdüse,
3. Aufnahme des Druckverlaufs infolge Kraftstoffzündung und Verbrennung,
4. Berechnung der Cetanzahl aus dem gemessenen Zündverzug.

Die Zeit zwischen Kraftstoffeinspritzung und -zündung, d. h. der Zündverzug wird möglichst genau gemessen. Dieser Zündverzug beträgt bei konventionellen Dieselkraftstoffen in einer Konstantvolumen-Apparatur typischerweise 3 bis 10 ms. Durch Messung von Kraftstoffen mit bekannter Cetanzahl unter gleichen Bedingungen kann die Apparatur kalibriert werden.

Die bekannten Konstantvolumen-Apparaturen weisen verschiedene Nachteile auf:
- Die Verwendung von mechanischen oder mechanisch-elektronischen Sensorelementen, die eine Auslösetoleranz von bis zu 2 ms aufweisen können, resultiert in einer mangelnden Präzision bei der Zündverzugsbestimmung. Durch eine Vielzahl von Messzyklen mit nachfolgender statischer Auswertung versucht man diesen Mangel zu beheben.
- Die Verwendung konventioneller Einspritzdüsen, die bei vergleichsweise geringen Drücken arbeiten, macht das Bestimmungsverfahren gegenüber Änderungen der Oberflächenspannung von sonst gleichen Kraftstoffproben empfindlich.
- Bei der Verwendung von konventionellen Pumpe-Düsen-Systemen ist die Kraftstoffeinspritzmenge nur ungenau bekannt und kann beträchtlich schwanken. Als Folge davon beobachtet man auch statistische Schwankungen des Zündverzugs.
- Die Verwendung von nichtthermostatisierten Einspritzdüsen verursacht eine Drift der Messergebnisse, bis die Einspritzdüse das thermische Gleichgewicht erlangt hat.
- Bei der Messung mehrerer Kraftstoffproben ist kein automatisierter Versuchsablauf möglich.
- Bei der Kraftstoffförderung von der Ansaugseite bis zur Einspritzdüse weisen die bekannten Apparaturen konstruktionsbedingt vergleichsweise große Totvolumina auf. Somit sind für eine Cetanzahl-Bestimmung auch große Probenvolumina erforderlich.

Aufgabe der vorliegenden Erfindung ist es daher, eine bessere und genauere Möglichkeit der Cetanzahlbestimmung mit einer Konstantvolumen-Apparatur zu schaffen.

Diese Aufgabe wird gemäß der Erfindung durch die im unabhängigen Vorrichtungsanspruch angegebene Apparatur und das im unabhängigen Verfahrensanspruch angegebene Verfahren gelöst. Vorteilhafte Ausgestaltungen der Erfindung sind Gegenstand der Unteransprüche.

Mit der Erfindung wird eine automatisierte Cetanzahlbestimmung mit hoher Genauigkeit und einem Höchstmaß an Reproduzierbarkeit erreicht, wie das bei den bekannten Konstantvolumen-Apparaturen und den damit durchgeführten Verfahren nicht möglich ist.

Ein Ausführungsbeispiel der Erfindung wird nachstehend unter Bezugnahme auf die anliegende schematische Zeichnung beschrieben, die in Gestalt eines Blockdiagramms eine Apparatur nach der Erfindung zeigt.

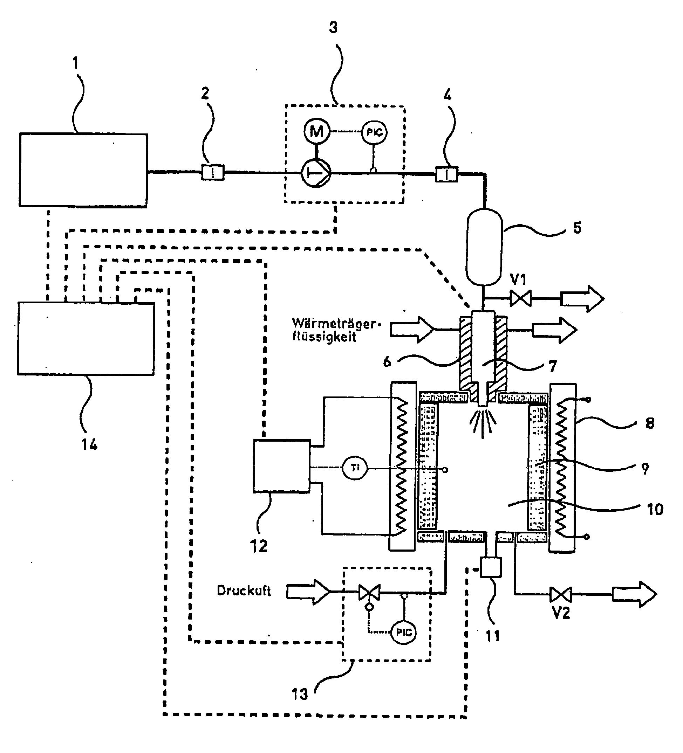

Die in der Zeichnung dargestellte erfindungsgemäße Apparatur umfasst einen Probengeber 1 zur automatisierten Probenzufuhr, einen ersten Filter 2, eine Förderpumpe 3, einen zweiten, der Förderpumpe nachgeschalteten Filter 4, einen Kraftstoffdruckspeicher 5, ein Hochdruckventil V1, einen Kühlmantel 6, eine Einspritzdüse 7, eine mit einer Brennkammerheizung 8 ausgestattete Brennkammer 9 mit einem Brennkammerinnenraum 10, ein Abgasventil V2, einen Druckaufnehmer 11 zum Aufnehmen des Brennkammerinnendrucks, einen Temperaturregler 12 für die Brennkammer, einen Druckregler 13 für die Brennkammer und einen Rechner 14.

Die automatisierte Probenzuführung aus dem Probengeber 1 erfolgt, indem aus einem bestimmten Probengefäß Kraftstoff mittels der Förderpumpe 3 angesaugt wird. Dazu wird rechnergesteuert aus einer Vielzahl vorhandener Probengefäße im Probengeber 1 ein Gefäß ausgewählt und die Ansaugleitung zur Förderpumpe in das ausgewählte Probengefäß eingetaucht. Solche automatisierten Probenzuführungen sind als sogenannte "Autosampler" bekannt und in verschiedensten Ausführungsformen kommerziell erhältlich. Alternativ kann auch ein elektrisch ansteuerbares Schaltventil verwendet werden. Dabei ist die Ansaugleitung der Förderpumpe 3 mittels eines Schaltventils gleichzeitig an mehrere Probengefäße angeschlossen. Über das Schaltventil kann dann rechnergesteuert eine bestimmte Probe angesaugt werden. Bei einem konkreten Ausführungsbeispiel werden beispielsweise elektrisch angesteuerte HPLC-Eluentenauswahlventile der Firma Rheodyne verwendet.

Der erste Filter 2 dient dazu, die nachfolgende Förderpumpe 3 vor Fehlfunktionen zu schützen. In der Praxis zeigt sich, daß Dieselkraftstoffproben unterschiedliche Schmutzpartikel, wie beispielsweise Rost oder Metallabrieb, enthalten können. Alternativ oder zusätzlich kann die in die Krafstoffprobe ragende Ansaugleitung mit einem Feinstfilter (Sintermetallfilter) ausgestattet werden.

Die Förderpumpe 3 ist eine ein/oder mehrstufige Kolbenverdichterpumpe, wie sie aus der instrumentellen Analytik bekannt ist. Solche in der HPLC-Analytik (HPLC High Performance Liquid Chromatography) gebräuchlichen Kolbenverdichterpumpen sind für die erfindungsgemäße Prüfapparatur geeignet, wenn sie bei einem Arbeitsdruck von mindestens 400 bar eine Flussrate >2 ml/min bereitstellen.

Bevorzugt werden pulsationsarme Doppelkolbenpumpen mit integrierter Druckregelung, die wenigstens 1600 bar Enddruck erzeugen können. Bei einem konkreten Ausführungsbeispiel wird das Fabrikat "Synova" (Vertrieb über Firma Techlab, Erkerode) verwendet. Die Förderpumpe 3 saugt den Kraftstoff an und erhöht den Druck auf ein für ein Piezo-Einspritzdüse 7 gebräuchliches Niveau, typischerweise 800 bis 2000 bar.

Nicht bevorzugt werden für die erfindungsgemäße Prüfapparatur Hochdruckpumpen, wie sie im Dieselmotorenbau verwendet werden, weil sie ein zu großes Totvolumen aufweisen.

Der zweite Filter 4 soll Schmutzteilchen oder andere Partikel zurückhalten und damit das Einspritzdüse 7 vor Fehlfunktionen schützen. Als Filterelemente kommen vorzugsweise Stahlfiltergewebe in Frage. Vorzugsweise wird ein Kraftstofffilter mit einem geringen Innenvolumen gewählt, so daß das Totvolumen der Druckleitung gering bleibt.

Der Kraftstoffdruckspeicher 5 ist mit einer möglichst kurzen Druckleitung mit der Einspritzdüse 7 verbunden. Das Speichervolumen wird so gewählt, daß genügend Krafstoff für wenigstens einen Einspritzzyklus ohne merklichen Druckabfall bereitgestellt wird. Ein weiteres Optimierungskriterium liegt in der Minimierung des Spülvolumens der Gesamtapparatur. So wird bei der Vermessung mehrerer Krafstoffproben nach Abschluß der Vermessung einer Kraftstoffprobe die gesamte Apparatur mit der neu zu bestimmenden Kraftstoffprobe gespült. Bei Verwendung eines kleinvolumigen Druckspeichers benötigt man für diesen Spülvorgang weniger Zeit und weniger Spülkraftstoff.

An die Verbindungsleitung zwischen dem Druckspeicher 5 und der Piezo-Einspritzdüse 7 ist ein Hochdruckventil V1 zum Spülen der Apparatur angeschlossen. Es wird rechnergesteuert geöffnet, wenn die Apparatur mit einer neuen Kraftstoffprobe befüllt wird.

Für die Wiederholgenauigkeit der Cetanzahlbestimmung ist es wichtig, daß der Kraftstoff in der Einspritzdüse 7 möglichst bis in die Düsenspitze eine konstante und definierte Temperatur aufweist. Dadurch wird sichergestellt, daß eine Kraftstoffprobe bei wiederholter Einspritzung eine gleichbleibende Viskosität aufweist.

Die Einspritzdüse 7 wird im unteren Teil vorzugsweise bis dicht an die Mehrlochdüsen mit einem selbsttragenden Kühlmantel 6 ummantelt. Der Kühlmantel 6 enthält im Inneren Bohrungen und Hohlräume, die mit einer thermostatisierten Wärmeträgerflüssigkeit, bevorzugt Wasser, druckbeaufschlagt durchflossen werden. Durch diesen Kühlmantel kann die Einspritzdüse 7 trotz heißer Brennkammer auf einer definierten Temperatur gehalten werden. Der Kühlmantel ist mechanisch lösbar mit der Brennkammer verbunden, und die Einspritzdüse 7 ist mit dem Kühlmantel 6 lösbar verbunden.

Die Einspritzdüse 7 ist ein handelsüblicher piezoelektrischer Injektor mit einer Mehrlochdüse für den Einsatz in Dieselmotoren. Der Piezo-Injektor zerstäubt unter Hochdruck den Kraftstoff in die Brennkammer 9. Der Einspritzdruck beträgt je nach gewählten Versuchsbedingungen zwischen 400 und 2000 bar.

Mittels solcher Piezo-Injektoren ist es möglich, zu einem bestimmten Zeitpunkt über eine definierte Zeitdauer eine genau definierte Kraftstoffmenge unter hohem Druck zu verstäuben.

Dien Einspritzdüse 7 ist mit dem Rechner 14 verbunden. Dieser enthält einen Signalgenerator mit nachgeschalteter analoger Präzisions-Stromendstufe, um dem Piezo-Injektor beim Einspritzvorgang eine definierte Ladungsmenge aufzuprägen.

Der zylindrischen Brennkammer 9 ist eine vorzugsweise als elektrische Widerstandsheizung ausgebildete Brennkammer 8 zugeordnet, die eine gleichmäßige Beheizung der gesamten Zylinderwandung sicherstellen soll.

Die elektrische Heizung ist mit einer äußeren Wäremisolation umgeben, um die Wärmeverluste der Apparatur zu minimieren.

Die elektrische Brennkammerheizung 8 ist mit einem autonom arbeitenden elektronischen Temperaturregler 12 verbunden, der einen elektrischen Leistungssteller enthält. Der Temperaturregler 12 besitzt ein oder mehrere Temperatursensoren, die die Ist-Temperatur im Brennkammerinnenraum und/oder in der Brennkammerwand feststellen. Durch diesen autonom arbeitenden Regelmechanismus kann die Lufttemperatur im Brennkammerinnenraum auf eine vorbestimmte feste Temperatur mit einer Toleranz <1°C eingestellt werden.

Der Temperaturregler 12 ist mit dem Rechner 14 verbunden, der den Sollwert übermittelt und die Ist-Werte abfragt.

Die zylindrische druckfeste Brennkammer 9 hat ein Innenvolumen von 500 bis 1200 ml, vorzugsweise von 600 bis 800 ml.

Als Werkstoffe für die Brennkammer eignen sich warmfeste, aufkohlungsbeständige Stähle, die den vollen möglichen Arbeitsbereich der Prüfapparatur wie den Kammerdruck von 10 bis 100 bar und die Lufttemperatur von 300 bis 730°C abdecken.

Die Brennkammer besteht wahlweise aus den Untereinheiten zylindrischer Brennkammertopf und lösbarer, kreisförmiger Bodenplatte, oder Brennkammerzylinder und lösbarer, kreisförmiger Kopf- und Bodenplatte.

Der Brennkammerinnenraum 10 ist über das rechnergesteuerte Abgasventil V 2 mit der Atmosphäre verbunden. Es wird zum Ende eine Messzyklus geöffnet, um die Verbrennungsabgase abzulassen.

Beim Befüllen der Brennkammer mit Druckluft erfolgt die Druckregelung mittels eines autonom arbeitenden Druckreglers 13, der vom Rechner 14 den Sollwert erhält und den Ist-Wert dem Rechner zurückübermittelt.

Eine hochdynamischer Quarz-Druckaufnehmer 11 zur Registrierung schneller Druckanstiege ist wahlweise brennraumbündig oder zurückversetzt mit dem Brennkammerinnenraum 10 verbunden. Der Druckaufnehmer 11 dient zur Bestimmung des Zeitpunkts der Kraftstoffzündung und ist mit dem Rechner 14 verbunden. Dieser enthält einen Transientrecorder mit Vorverstärker zur Signalkonditionierung des Ladungssignals des Quarz-Druckaufnehmers. Dabei wird die aus den Komponenten Quarz-Druckaufnehmer - Vorverstärker - Transientenrecorder bestehende Messkette so aufeinander abgestimmt, daß bei der Digitalisierung des Drucksignals eine Zeitauflösung von vorzugsweise besser als 10 µs erreicht wird. Der Druckaufnehmer 11 besitzt einen Kühlmantel, der von Wärmeträgerflüssigkeit, vorzugsweise Wasser durchflossen wird.

Alle Arbeits- und Messschritte eines Messzyklus werden mittels Software durch den Rechner 14 gesteuert und laufen automatisiert ab.

Bei der Apparatur werden die hochdruckseitigen Verbindungsleitungen vom Auslaß der Förderpumpe 3 bis zum Einlaß der Einspritzdüse 7 vorzugsweise mit druckfesten Edelstahlkapillarleitungen ausgeführt, wie sie in der HPLC-Technik üblich sind. Dadurch wird das Totvolumen gering gehalten. Alle kraftstoffberührenden Teile einschließlich Probengeber 1 und Förderpumpe 3 können auch gesondert thermostatisiert werden. Dadurch ist eine Cetanzahl-Bestimmung auch bei viskosen Dieselkraftstoffen, wie beispielsweise Pflanzenölen, möglich.

Die beschriebene erfindungsgemäße Apparatur weist eine Reihe von Vorteilen gegenüber dem Stand der Technik auf:
- Eine Cetanzahl-Bestimmung ist auch bei geringen Probenmengen möglich, weil die Apparatur nur ein geringes Totvolumen aufweist.
- Wegen der Anwendung der hohen Einspritzdrücke haben unterschiedliche Oberflächenspannungen bei sonst gleichen Kraftstoffproben keine störenden Auswirkungen.
- Es ist ein automatisierter unbeaufsichtigter Betrieb möglich.
- Durch exakte Kontrolle der Kraftstofftemperatur, der Brennkammerinnentemperatur und des Brennkammerinnendrucks, des Einspritzzeitpunkts und der Einspritzdauer erhält man sehr gute Präzision und Wiederholbarkeit bei der Cetanzahl-Bestimmung.

Zu den typischen gewerblichen Anwendungsgebieten der erfindungsgemäßen Apparatur gehören beispielsweise
- die kontinuierliche Cetanzahl-Bestimmung bei der Herstellung von Raffinerieerzeugnissen,
- die Cetanzahl-Bestimmung von Mitteldestillaten in Auftragslaboratorien,
- die Cetanzahl-Bestimmung von Sonderkraftstoffen, wie beispielsweise GTL-Kraftstoffen,
- die Cetanzahl-Bestimmung von Mischungen aus fossilem Dieselkraftstoff und biogenen Kraftstoffen,
- die Untersuchung der Wirkung von Kraftstoffaditiven, wie beispielsweise Zündbeschleunigem,
- Untersuchungen in Schadensfällen, wenn nur geringe Kraftstoffmengen zur Verfügung stehen.

Die automatisierte Bestimmung der Cetanzahl mittels einer Apparatur nach der Erfindung erfolgt im wesentlichen mit folgenden Verfahrensschritten:

| | |
|---|---|
| S1: | Auswahl einer Kraftstoffprobe. Im Rechner 14 ist eine Liste aller Kraftstoffproben und deren Lagerplätze im Probengeber 1 gespeichert. Programmgesteuert wird eine Probe ausgewählt und die Ansaugleitung der Förderpumpe 3 rechnergesteuert mit dem Lagerplatz dieser Probe verbunden. |
| | |
| S2: | Befüllung der Apparatur mit Kraftstoff. Bei geöffnetem Hochdruckventil V1 saugt die Förderpumpe 3 die gewählte Probe an und fördert so lange, bis das Leitungsvolumen gespült ist. Anschließend wird das Hochdruckventil V1 wieder geschlossen. Zusätzlich kann es anschließend erforderlich sein, die Einspritzdüse 7 mit Drücken < 50 bar durchzuspülen. |
| | |
| S3: | Kraftstoff-Druckaufbau. Die Förderpumpe 3 erhält vom Rechner 14 den Einspritzdruck-Sollwert, der dann autonom angefahren wird. |
| | |
| S4: | Einstellung eines definierten Luftdrucks in der Brennkammer. Der Luftdruck 13 erhält vom Rechner 14 den Luftdruck-Sollwert im Brennkammerinnenraum 10. Bei geschlossenem Abgasventil V2 wird dieser dann autonom eingestellt. |
| | |
| S5: | Einstellung einer definierten Brennkammerinnenraum-Temperatur. Der Temperaturregler 12 erhält vom Rechner 14 den Temperatur-Sollwert für den Brennkammrinnenraum 10. Dieser wird dann autonom eingestellt. |
| | |
| S6: | Bestimmung der Zündverzugszeit. Der Rechner 14 überprüft durch Abfrage der autonom arbeitenden Regelorgane (Förderpumpe 3, Temperaturregler 12 und Druckregler 13), ob alle Soll-Werte innerhalb vordefinierter Grenzen eingehalten werden. Anschließend wird durch den Rechner 14 der Einspritzvorgang ausgelöst. Dabei wird ein definiertes Stromsignal so an die als Piezo-Injektor ausgebildete Einspritzdüse 7 geschickt, daß die Einspritzdauer und der Ventilhub vorgegebene Werte erreichen. Der zeitliche Beginn der Einspritzung wird durch den Rechner aufgezeichnet. Gleichzeitig registriert der Rechner 14 über den Druckaufnehmer 11 den zeitlichen Druckverlauf im Brennkammerinnenraum 10. Die Zeitdifferenz zwischen Einspritzbeginn und Beginn des Druckanstiegs wird als Zündverzugszeit zur Berechnung der Cetanzahl herangezogen. |
| | |
| S7: | Abblasen des Abgases. Der Rechner 14 unterbricht über den Druckregler 13 die Gaszufuhr zum Brennkammerinnenraum 10 und öffnet das Abgasventil V2. |

Die Schritte S3 bis S7 werden wenigstens einmal wiederholt. Aus den Einzelergebnissen wird dann eine mittlere Cetanzahl für eine Kraftstoffprobe berechnet. Danach wird, beginnend mit der Auswahl einer neuen Kraftstoffprobe, der beschriebene Verfahrensablauf wiederholt.

Die dargestellten Verfahrensschritte werden sequentiell ausgeführt. Ein Teil der Verfahrensschritte wie beispielsweise die Schritte S3, S4 und S5, können auch zeitlich parallel ausgeführt werden.

## Patentansprüche

1. Apparatur zur Cetanzahlbestimmung, mit
einer Konstantvolumen-Brennkammer (9) mit einer Einspritzdüse (7),
Mitteln (13) zum Befüllen der Brennkammer (9) mit Druckluft, und Mitteln (8, 12) zum Aufheizen der Brennkammer,
Mitteln (11, 14) zur Aufnahme des Druckverlaufs in der Brennkammer (9) während der Zündung und Verbrennung von eingespritztem Kraftstoff,
**dadurch gekennzeichnet, daß**
die Einspritzdüse (7) als temperaturgesteuerter Piezo-Injektor ausgebildet ist, die aus einem automatischen Probengeber (1) über eine Hochdruckpumpe (3) und einen Kraftstoffhochdruckspeicher (5) mit Kraftstoffproben gespeist wird.

2. Apparatur nach Anspruch 1, wobei der automatische Probengeber (1) eine Mehrzahl von wahlweise zugreifbaren Probengefäßen enthält.

3. Apparatur nach Anspruch 1 oder 2, wobei die Einspritzdüse (7) über einen sie umgebenden Kühlmantel (6) temperaturgesteuert wird, der von einer thermostatisierten Flüssigkeit durchflossen ist.

4. Apparatur nach Anspruch 3, wobei der Kühlmantel (6) selbsttragend und abnehmbar an der Brennkammer (9) angeordnet ist.

5. Apparatur nach einem der Ansprüche 1 bis 4, wobei die Hochdruckpumpe eine Kolbenverdichterpumpe mit integrierter Druckregelung ist.

6. Apparatur nach einem der Ansprüche 1 bis 5, wobei der Einspritzdruck der Kraftstoffproben in die Brennkammer 400 bis 2000 bar beträgt.

7. Apparatur nach einem der Ansprüche 1 bis 5, wobei der Einspritzdruck der Kraftstoffproben in die Brennkammer 800 bis 2000 bar beträgt.

8. Apparatur nach einem der Ansprüche 1 bis 7, wobei der Brennkammer (9) eine den Brennkammermantel gleichmäßig umgebende elektrische Widerstandsheizung (8) und ein Temperaturregler (12) zugeordnet sind.

9. Apparatur nach einem der Ansprüche 1 bis 8, wobei der Brennkammer (9) ein Quarz-Druckaufnehmer (11) zur Aufnahme des Druckverlaufs in der Brennkammer dient.

10. Apparatur nach einem der Ansprüche 1 bis 9, mit einem Rechner (14) zur Steuerung der Komponenten der Apparatur, der Betriebsparameter und der Probenabgabe.

11. Verfahren zur Cetanzahlbestimmung unter Verwendung einer Apparatur nach einem der Ansprüche 1 bis 10, mit folgenden Schritten:
a) Auswahl einer Kraftstoffprobe,
b) Fördern der Kraftstoffprobe und Druckaufbau der Kraftstoffprobe bis auf den gewünschten Einspritzdruck,
c) Einstellung eines definierten Luftdrucks in der Brennkammer und Einstellung einer definierten Brennkammer-Innentemperatur,
d) Auslösen des Einspritzvorgangs und Aufnahme des Druckverlaufs in der Brennkammer, und Bestimmen der Cetanzahl aus dem Druckverlauf,
e) Abblasen des Abgases aus der Brennkammer.

12. Verfahren nach Anspruch 11, wobei die Schritte b) bis e) ein- oder mehrfach wiederholt werden.

## Claims

1. Apparatus for determining the cetane number, comprising
a constant-volume combustion chamber (9) with an injection nozzle (7), means (13) for filling the combustion chamber (9) with compressed air, and means (8, 12) for heating the combustion chamber,
means (11, 14) for measuring the pressure curve in the combustion chamber (9) during the ignition and combustion of injected fuel,
**characterized in that**
the injection nozzle (7) is embodied as a temperature-controlled piezo injector which is supplied with fuel samples from an automatic sample supplier (1) via a high-pressure pump (3) and a high-pressure fuel reservoir (5).

2. Apparatus according to claim 1, wherein the automatic sample supplier (1) contains a plurality of selectively accessible sample containers.

3. Apparatus according to claim 1 or 2, wherein the injection nozzle (7) is temperature-controlled via a cooling sleeve (6) which surrounds it and through which a thermostatised liquid flows.

4. Apparatus according to claim 3, wherein the cooling sleeve (6) is self-supporting and removably disposed on the combustion chamber (9).

5. Apparatus according to one of claims 1 to 4, wherein the high-pressure pump is a piston compressor pump with integrated pressure control.

6. Apparatus according to one of claims 1 to 5, wherein the pressure at which the fuel samples are injected into the combustion chamber is 400 to 2,000 bar.

7. Apparatus according to one of claims 1 to 5, wherein the pressure at which the fuel samples are injected into the combustion chamber is 800 to 2,000 bar.

8. Apparatus according to one of claims 1 to 7, wherein an electric resistance heater (8), which uniformly surrounds the combustion chamber sleeve, and a temperature controller (12) are associated with the combustion chamber (9).

9. Apparatus according to one of claims 1 to 8, wherein a quartz pressure sensor (11) serves the combustion chamber (9) for measuring the pressure curve in the combustion chamber.

10. Apparatus according to one of claims 1 to 9, comprising a computer (14) for controlling the components of the apparatus, the operating parameters and the dispensing of samples.

11. Method for determining the cetane number using an apparatus according to one of claims 1 to 10, including the following steps:
a) selecting a fuel sample,
b) conveying the fuel sample and building up the pressure of the fuel sample to the desired injection pressure,
c) setting a defined air pressure in the combustion chamber and setting a defined combustion chamber internal temperature,
d) triggering the injection process and recording the pressure curve in the combustion chamber, and determining the cetane number from the pressure curve,
e) releasing the waste gas from the combustion chamber.

12. Method according to claim 11, wherein steps b) to e) are repeated once or several times.

## Revendications

1. Appareil destiné à déterminer l'indice de cétane, comprenant
une chambre de combustion (9) à volume constant dotée d'un injecteur (7), des moyens (13) permettant de remplir la chambre de combustion (9) en air comprimé, et des moyens (8, 12) permettant de chauffer la chambre de combustion,
des moyens (11, 14) permettant de mesurer l'évolution de la pression dans la chambre de combustion (9) pendant l'allumage et la combustion du carburant injecté,
**caractérisé en ce que**
l'injecteur (7) est réalisé sous la forme d'un injecteur piézoélectrique commandé par la température et est alimenté en échantillons de carburant à partir d'un échantillonneur automatique (1) par l'intermédiaire d'une pompe haute pression (3) et d'un réservoir haute pression de carburant (5).

2. Appareil selon la revendication 1, l'échantillonneur automatique (1) contenant une pluralité de récipients d'échantillons accessibles au choix.

3. Appareil selon la revendication 1 ou 2, l'injecteur (7) étant commandé par la température par l'intermédiaire d'une enveloppe de refroidissement (6) qui l'entoure et est traversée par un liquide thermostatisé.

4. Appareil selon la revendication 3, l'enveloppe de refroidissement (6) étant autoporteuse et disposée de façon amovible sur la chambre de combustion (9).

5. Appareil selon l'une quelconque des revendications 1 à 4, la pompe haute pression étant une pompe de compresseur à piston avec régulation de pression intégrée.

6. Appareil selon l'une quelconque des revendications 1 à 5, la pression d'injection des échantillons de carburant dans la chambre de combustion allant de 400 à 2000 bars.

7. Appareil selon l'une quelconque des revendications 1 à 5, la pression d'injection des échantillons de carburant dans la chambre de combustion allant de 800 à 2000 bars.

8. Appareil selon l'une quelconque des revendications 1 à 7, un chauffage à résistance électrique (8) entourant uniformément l'enveloppe de la chambre de combustion et un régulateur de température (12) étant associés à la chambre de combustion (9).

9. Appareil selon l'une quelconque des revendications 1 à 8, un capteur de pression à quartz (11) servant à la chambre de combustion (9) pour mesurer l'évolution de la pression dans la chambre de combustion.

10. Appareil selon l'une quelconque des revendications 1 à 9, comprenant un ordinateur (14) pour la commande des composants de l'appareil, des paramètres de service et de la livraison des échantillons.

11. Procédé de détermination de l'indice de cétane en utilisant un appareil selon l'une quelconque des revendications 1 à 10, comportant les étapes suivantes :
a) choix d'un échantillon de carburant,
b) transport de l'échantillon de carburant et montée en pression de l'échantillon de carburant jusqu'à la pression d'injection voulue,
c) réglage d'une pression d'air définie dans la chambre de combustion et réglage d'une température intérieure définie de la chambre de combustion,
d) déclenchement du processus d'injection et mesure de l'évolution de la pression dans la chambre de combustion et détermination de l'indice de cétane à partir de l'évolution de la pression,
e) purge des gaz d'échappement de la chambre de combustion.

12. Procédé selon la revendication 11, les étapes b) à e) étant répétées une ou plusieurs fois.
